# EUROPEAN PATENT SPECIFICATION

(11) **EP 2 482 869 B1**
(45) Date of publication and mention of the grant of the patent: **25.09.2019**
(21) Application number: 10770662.4
(22) Date of filing: 29.09.2010
(51) Int. Cl.: A61M 13/00

(54) **MULTIMODAL SURGICAL GAS DELIVERY SYSTEM FOR LAPAROSCOPIC SURGICAL PROCEDURES**
MULTMODALES CHIRURGISCHES GASVERABREICHUNGSSYSTEM FÜR LAPAROSKOPISCHE EINGRIFFE
SYSTÈME MULTIMODAL D'APPORT DE GAZ CHIRURGICAL POUR INTERVENTIONS CHIRURGICALES LAPAROSCOPIQUES

(30) Priority: 29.09.2009 US 246921 P
(43) Date of publication of application: 08.08.2012
(73) Proprietor: SurgiQuest, Incorporated, Orange, CT 06477 (US)
(72) Inventor: STEARNS, Ralph, Bozrah, CT 06334 (US); FELDMAN, Dennis, Apollo Beach, FL 33572 (US); TANG, Raymond, Yue-Sing, CT 06513 (US)
(74) Representative: Hoefer & Partner Patentanwälte mbB
(86) International application number: PCT/US2010/050688
(87) International publication number: WO 2011/041387

(56) References cited:
- WO-A1-2009/137693
- US-A1- 2007 088 275
- US-A1- 2007 244 363
- US-A1- 2009 137 943

## Description

### BACKGROUND

### Field of the Invention

The present invention relates to surgical insufflation systems, and surgical smoke evacuation systems. Particularly, the present invention is directed to a multimodal system capable of surgical insufflation, smoke evacuation and recirculation of insufflation gasses.

### Description of Related Art

Laparoscopic, or "minimally invasive" surgical techniques are becoming increasingly more common. Benefits of such procedures include reduced trauma to the patient, reduced opportunity for infection, and decreased recovery time. Such procedures within the abdominal (peritoneal) cavity are typically performed through a device known as a trocar or cannula, which facilitates the introduction of laparoscopic instruments into the abdominal cavity of a patient.

Additionally, such procedures commonly involve filling or "insufflating" the abdominal (peritoneal) cavity with a pressurized fluid, such as carbon dioxide, to create what is referred to as a pneumoperitoneum. The insufflation can be carried out by a surgical access device (sometimes referred to as a "cannula" or "trocar") equipped to deliver insufflation fluid, or by a separate insufflation device, such as an insufflation (veress) needle. Introduction of surgical instruments into the pneumoperitoneum without a substantial loss of insufflation gas is desirable, in order to maintain the pneumoperitoneum.

During typical laparoscopic procedures, a surgeon makes three to four small incisions, usually no larger than about twelve millimeters each, which are typically made with the surgical access devices themselves, typically using a separate inserter or obturator placed therein. Following insertion, the inserter is removed, and the trocar allows access for instruments to be inserted into the abdominal cavity. Typical trocars often provide means to insufflate the abdominal cavity, so that the surgeon has an open interior space in which to work.

The trocar must provide a means to maintain the pressure within the cavity by sealing between the trocar and the surgical instrument being used, while still allowing at least a minimum freedom of movement of the surgical instruments. Such instruments can include, for example, scissors, grasping instruments, occluding instruments, cauterizing units, cameras, light sources and other surgical instruments. Sealing elements or mechanisms are typically provided on trocars to prevent the escape of insufflation gas. Sealing elements or mechanisms typically include a duckbill-type valve made of a relatively pliable material, to seal around an outer surface of surgical instruments passing through the trocar.

Further, in laparoscopic surgery, electrocautery an other techniques (e.g. harmonic scalpels) create smoke and other debris in the surgical cavity, reducing visibility by fogging the view from, and coating surfaces of endoscopes and the like.

A variety of surgical insufflation systems and smoke evacuation systems are known in the art. Additionally, SurgiQuest, Inc., Orange, CT USA has developed surgical access devices that permit access to an insufflated surgical cavity without conventional mechanical seals, and has developed related systems for providing sufficient pressure and flow rates to such access devices, as described in whole or in part in U.S. Patent Publication Number 2007/0088275, as well as in U.S. Patent Application Serial Number 61/104,448, filed October 10, 2008, for example. Further, US 2009/0137943 A1 discloses a system according to the preamble part of claim 1.

The present invention relates to multimodal systems, and related devices and methods, capable of performing multiple surgical gas delivery functions, including insufflation to standard or specialized surgical access devices or other instruments, such as veress needles and the like, smoke evacuation through standard or specialized surgical access devices, and specialized functions, such as recirculation and filtration of insufflation fluids, such as with the above-mentioned surgical access devices described in U.S. Patent Publication Number 2007/0088275, as well as those in U.S. Patent Numbers 7,182,752, 7,285,112, 7,413,559 or 7,338,473, for example.

Use of a single multimodal system such as those described herein reduces costs by requiring purchase of only one system while achieving multiple functions, and also thereby reduces the amount of equipment needed in an operating room, thus reducing clutter and allowing space for other necessary equipment.

### SUMMARY

The purpose and advantages of the present invention will be set forth in and apparent from the description that follows. Additional advantages of the invention will be realized and attained by the systems particularly pointed out in the written description and claims hereof, as well as from the appended drawings.

To achieve these and other advantages and in accordance with the purpose of the invention, as embodied, the invention includes, in one aspect, a multimodal surgical gas delivery system having a fluid pump, supply conduit, return conduit, adjustable back-pressure control valve, insufflation control and conduit, a pressure sensor and a conduit set. The fluid pump is adapted and configured to circulate insufflation fluid through the system. The supply conduit is in fluid communication with an output of the fluid pump and is configured and adapted for delivering pressurized insufflation fluid to an output port of the control unit. The return conduit is in fluid communication with an input of the fluid pump for delivering insufflation fluid to the fluid pump and is configured and adapted for returning insufflation fluid to an input port of the control unit. The adjustable back-pressure control valve is in fluid communication with the supply conduit and the return conduit, and is adapted and configured to respond to a supply conduit pressure exceeding a set pressure by opening and directing fluid from the supply conduit to the return conduit. The insufflation control controls addition of insufflation fluid into the system, from an insufflation gas source. The insufflation conduit delivers insufflation gas to the system from the insufflation control. The pressure sensor is adapted and configured to sense pressure of a surgical cavity through the insufflation conduit. The control panel is configured and adapted to permit a user to select a mode of the multimodal surgical gas delivery system. The conduit set is adapted and configured to connect to the supply, return and insufflation conduits, and to a plurality of surgical devices in fluid communication with the surgical cavity.

The subject systems further include a switching valve in connection with the supply, return and insufflation conduits, configured and adapted to divert the insufflation conduit between fluid connection with one or more of the surgical devices, and the return conduit to the fluid pump.

The insufflation conduit can serve as a conduit for detecting abdominal pressure.

The subject systems can further include a conduit for detecting abdominal pressure, separate from the insufflation conduit.

It is to be understood that both the foregoing general description and the following detailed description are exemplary and are intended to provide further explanation of the invention.

### BRIEF DESCRIPTION OF THE DRAWINGS

The accompanying drawings, which are incorporated in and constitute part of this specification, are included to illustrate and provide a further understanding of the systems, devices and related methods of the invention. Together with the description, the drawings serve to explain the principles of the invention, wherein:
Figure 1 is a schematic illustration of a multimodal surgical gas delivery system for laparoscopic surgical procedures;
Figure 2 is a schematic illustration of a multimodal surgical gas delivery system for laparoscopic surgical procedures; and
Figure 3 is a schematic illustration of a multimodal surgical gas delivery system for laparoscopic surgical procedures.

### DETAILED DESCRIPTION

Reference will now be made in detail to select embodiments of the invention, examples of which are illustrated in the accompanying drawings.

The systems presented herein may be used for surgical gas delivery, including insufflation, smoke evacuation, and/or recirculation in connection with suitable surgical devices, and in applicable surgical procedures. The present invention is particularly suited for minimizing the amount of equipment needed in a surgical operating room (operating theater), in that the subject systems are capable of performing multiple functions, and therefore also allow flexibility of surgical technique.

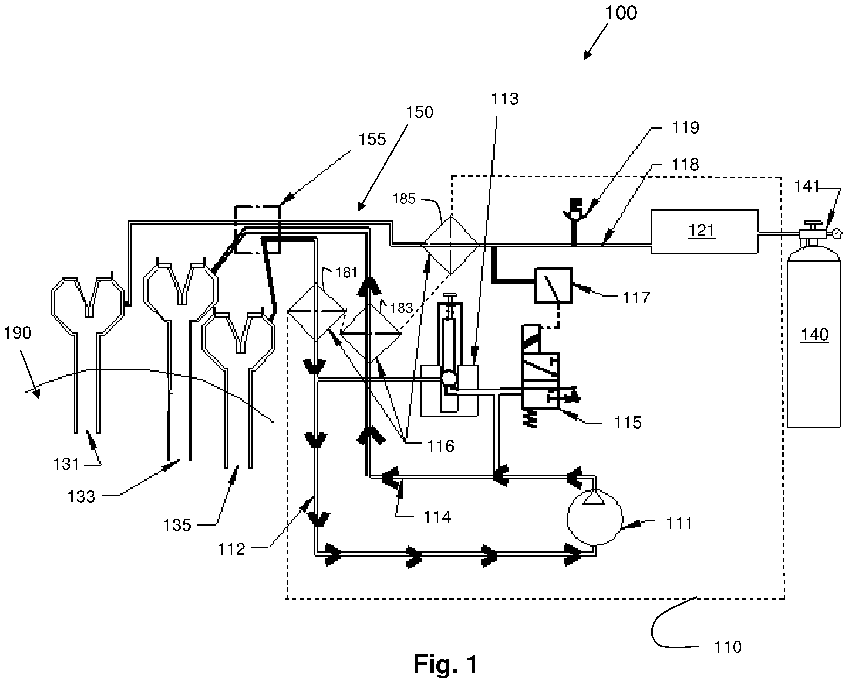

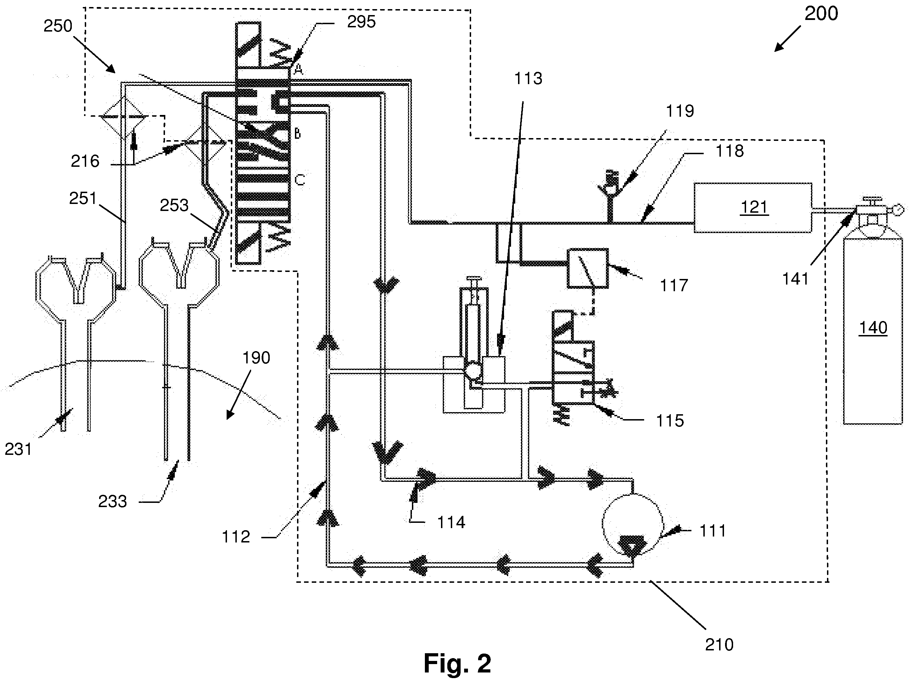

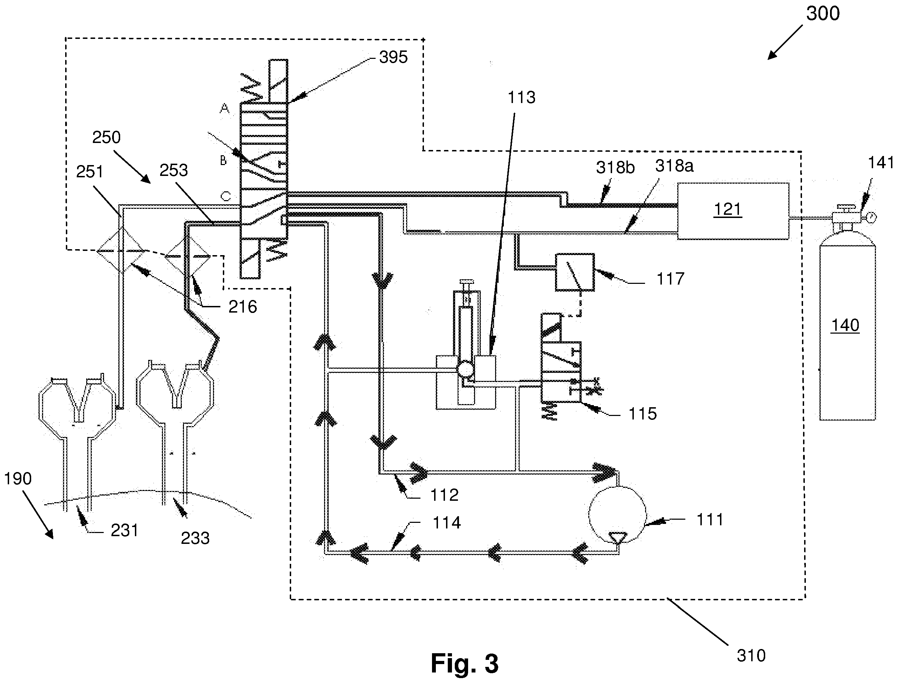

Figures 1-3 illustrate different systems. The system 100 of Figure 1 illustrates a multimodal system adapted and configured to operate in any selected mode using three surgical devices that are in fluid connection with a surgical cavity 190 (e.g. a patient's abdominal cavity). The system 200 of Figure 2 and system 300 of Figure 3 are each adapted and configured to operate in any selected mode using two surgical devices in fluid communication with a surgical cavity 190. In any case, it is conceived that additional surgical devices can be employed in parallel, for performing duplicative or still additional functions.

Such surgical devices can be any desired device capable of permitting fluid communication, including but not limited to standard surgical access devices (e.g. trocars, cannulas), veress needles, and the like. It is conceived that the subject systems can alternatively or additionally be adapted and configured to interface with a main lumen of such access devices by mounting to the proximal end portion thereof, or alternatively by a fluid conduit placed through the lumen of the access device. Such an arrangement may be desirable in cases where a volumetric flow of fluid to be passed through the system exceeds a capacity of an insufflation port and/or stopcock on a standard surgical access device. Typically, surgical access devices are provided with a stopcock arranged in fluid communication with a space defined below a seal element to permit connection to an insufflator.

Systems described in U.S. Patent Publication Number 2007/0088275, as well as in U.S. Patent Application Serial Number 61/104,448, filed October 10, 2008, for example, provide pressurized gas to and remove depressurized gas from specialized surgical access devices, which penetrate into a surgical cavity, such as a patient's abdominal cavity. The access devices are adapted and configured to form a pressure barrier to inhibit losses of insufflation gas to the atmosphere. Gas from the abdomen interchanges with gas coming from the access device(s), a portion of which is collected and recycled through the system, and is re-pressurized, passing through one or more filters along the way. During this recycling process, smoke and/or other circulating debris, such as atomized fluids, are removed by the filters improving visibility within the surgical cavity, thus aiding in the surgical procedure.

Systems of the present invention utilize the inherent smoke and debris clearing capability of the systems configured for use with the aforementioned surgical access devices to perform additional functions with conventional access devices, including insufflation and smoke evacuation.

As mentioned above, three main arrangements are described herein. The first arrangement enables smoke removal and insufflation using three conventional access devices. The second and third arrangements enable smoke removal and insufflation using only two conventional access devices.

As illustrated in Figure 1, a multimodal surgical gas delivery system 100 includes a fluid pump 111 adapted and configured to circulate insufflation fluid through the system 100. A supply conduit 114 is in fluid communication with an output of the fluid pump 111 and is configured and adapted for delivering pressurized insufflation fluid to an output port 183 of the control unit 110. A return conduit 112 is in fluid communication with an input of the fluid pump 111 for delivering insufflation fluid to the fluid pump 111, and is configured and adapted for returning insufflation fluid to an input port 181 of the control unit 110.

An adjustable back-pressure control valve 113 is provided in fluid communication with the supply conduit 114 and the return conduit 112, and is adapted and configured to respond to a supply conduit pressure exceeding a set pressure, by opening and directing fluid from the supply conduit 114 to the return conduit 112. The back-pressure control valve 113 can be a mechanical valve, such as a resiliently-biased valve. Alternatively, the back-pressure control valve 113 can be an electro-mechanical valve, responding to a high pressure signal from one or more pressure sensors (e.g. 117) within the system 100.

An insufflation subunit 121 is provided and is adapted and configured to receive a supply of insufflation gas (e.g. carbon dioxide) from a source 140 (e.g. a local tank or central distribution system), which may also pass through a pressure regulator 141 prior to entering the system 100. The insufflation subunit 121 is connected through an insufflation conduit 118 for delivering insufflation gas to the rest of the system 100 from the insufflation subunit 121, and includes a pressure sensor (not illustrated separately) adapted and configured to sense pressure of a surgical cavity 190 through the insufflation conduit, and an insufflation control (not illustrated separately), for controlling (as by stopping and starting) addition of insufflation fluid into the system 100, from the source 140.

Further, the system 100, or any system described herein, is controlled by a user through a control panel, such as one provided on or otherwise in connection with the control unit 110. Such control panel is preferably adapted and configured to permit a user to select a mode for the multimodal surgical gas delivery system, such as by way of a switch, touch screen or other user interface, such as a graphical user interface (GUI) that permits flexibility of the unit 110, while reducing clutter and/or confusion due to excess controls, permitting only selection from a predetermined set of appropriate parameters in any given mode. After selecting a mode, such as insufflation only, smoke evacuation only, combined smoke evacuation and insufflation, recirculation only, or combined recirculation and smoke evacuation, for example, parameters that may be adjustable include, for example, flow rate (e.g. liters/minute), pressure (e.g. in mmHg), and conditioning parameters (e.g., temperature, humidity), and the like. As used herein, such "recirculation" mode, alone or combined with other modes, is one that is suitable for providing sufficient pressures and flow rates to drive surgical access devices such as those described in U.S. Patent Publication Number 2007/0088275, as well as in U.S. Patent Application Serial Number 61/104,448, filed October 10, 2008, and/or those described in U.S. Patent Numbers 7,182,752, 7,285,112, 7,413,559 or 7,338,473, for example.

A conduit set or tube set 150 is also preferably provided, and is adapted and configured to connect at one end to the supply 114, return 112 and insufflation 118 conduits, and at the opposing end to a plurality of surgical devices 131, 133, 135, provided in fluid communication with the surgical cavity 190. The configuration of the tube set 150 can vary, depending on the desired implementation, as mentioned above. In the case of the system 100 of Figure 1, the tube set 150 preferably has a unitary, multi-lumen connection to input 181, output 183 and insufflation 185 ports, and separate connections to individual surgical devices 131, 133, 135.

In one preferred aspect, the tube set 150 has a compound, multi-lumen tube, beginning at the connections to the ports 181, 183, 185 to the control unit 110 for a predetermined distance from the control unit 110, generally until about the distance between the control unit 110 and an operating table, at which point a furcation 155 yields multiple separate tubes. In the case of the system 100 of Figure 1, three separate tubes, separately lead to each of the surgical devices 131, 133, 135, which may be surgical access devices (e.g., cannulas, trocars) with insufflation capability, or other instruments, such one or more veress needles. The surgical devices are thus individually connected to one of the supply 114, return, 112 and insufflation 118 conduits, and therefore respectively facilitates that function. That is, the surgical devices 131, 133, 135 facilitate insufflation into and sensing of, supply filtered insufflation gas to, or remove contaminated gas from the abdominal cavity 190.

As set forth above, in one preferred aspect, the separate distal tube portions of the tube set 150 are connected by way of a conventional fitting, such as a luer-lock fitting on a conventional surgical device. The precise configuration of the tube set 150 can vary depending on the desired configuration. Moreover, as described for example, in U.S. Patent Application Serial Numbers US 11/960,701, filed December 20, 2007; US 61/104,448, filed October 10, 2008; and US 61/195,898, filed October 10, 2008, a unitary filter element can be provided to which the tube set 150 is connected. In such an arrangement, the filter(s) 116 is (are) arranged between the tube set 150 and the supply 114, return, 112 or insufflation 118 conduits, and is (are) provided with integral input 181, output 183 and insufflation 185 ports, which can be a unitary, multi-lumen connection, for example.

A filter 116 that is suitable for one application or function, may be suitable for use in another application or function. For example, a filter suitable for use in a recirculation function with specialized surgical access devices, such as Air Seal™ surgical cannulas, available from SurgiQuest, Inc., Orange, CT USA, as described in whole or in part in U.S. Pub. No. 2007/0088275 and US 61/104,448, filed October 10, 2008, for example, may be suitable for use in smoke evacuation and insufflation functions using conventional surgical access devices, requiring only a different, particular tube set 150. The above-mentioned SurgiQuest Air Seal™ surgical cannulas utilize relatively large flow rates compared to flow rates needed for smoke evacuation and/or insufflation, and therefore a filter suitable for large flow rates will likely be suitable for smaller flow rates.

Alternatively, a specially configured filter can be provided, with dimensions and other parameters tailored to flow rates of different functions. Moreover, the tube set 150 and filter 116 can be mutually configured as a set for a particular application and provided as a kit, and permanently connected, if desired.

If so desired, systems in accordance with the invention can be provided with capability to automatically identify consumables (e.g., filters and/or tube sets) used in connection with the system, such as by a radio-frequency identification (RFID) transponder, bar code, or other data-carrying element provided thereon. In such an arrangement, the system (e.g. 100) identifies the consumable and switches to the appropriate mode (e.g., recirculation, smoke evacuation, etc.).

The system 100 of Figure 1 includes an additional dump valve 115 in connection with the fluid supply conduit 114. In addition to the short-circuiting action of the back-pressure control valve 113 described above, the system 100 is provided with a pressure sensor 117, which can be mechanical but is, as illustrated, electronic. The pressure sensor 117, if provided, is in fluid communication with the insufflation conduit 118 or other source of abdominal pressure. When an over-pressure condition is sensed, the pressure sensor 117 signals the dump valve 115 to release fluid out of the system 100. As illustrated, the dump valve 115 is electro-mechanical, but alternatively may be fully mechanical, as desired.

One or more additional dump valves (e.g. dump valve 119) can be provided to reduce any possibility of overpressure conditions, and/or to provide redundancy for other safety features.

In the embodiment of Figure 1, the system 100 operates, as set forth above, with one surgical device 131 being used for insufflation and sensing functions, another surgical device 135 serving to remove insufflation gas from the abdomen, which then passes through a filter, such as an ultralow-penetration air ("ULPA") filter element 116 for example, before returning to the pump 111. The filter 116 is preferably configured and adapted to clear all or essentially all smoke and debris from the gas passing therethrough, with the gas being returned to the abdominal cavity 190 through a third surgical device 133. As illustrated, another filter element 116 can be provided in connection with the supply conduit 114 leading from the pump 111.

For the purposes of explanation and illustration, and not limitation, a schematic illustration of an exemplary embodiment of a surgical gas delivery system in accordance with another aspect of the invention is shown in Fig. 2 and is designated generally by reference character 200. As compared with the system 100 of Figure 1, the system 200 only requires two surgical devices (e.g., cannulas). The functionalities of components described above in connection with the system 100 of Figure 1 are the same as the corresponding components of the system 200 of Figure 2, unless otherwise specified.

The system 200 is in many ways similar to the system 100 of Figure 1, but with the addition of a diverting valve 295, having three conduits 112, 114, 118 leading from other active internal system components, and two conduits 251, 253 leading, respectively, to two different surgical devices 231, 233.

As illustrated, the diverting valve 295 is provided integrally, within the control unit 210, as indicated schematically by placement of broken line referenced by number 210. The diverting valve is provided with three positions - positions, A, B and C, corresponding to different functions, as described below. When the sensing function of the system 200 is active, the diverting valve 295 is positioned in, as illustrated, position "A", permitting connection of the insufflation/sensing conduit 118 therethrough, through one or both of the conduits 251, 253 of the tube set 250, and to one or both of the surgical devices 231, 233. If configured to connect the insufflation/sensing conduit 118 to more than one surgical device, the potential for a lumen of such surgical device being blocked and thus not providing an accurate reading, is reduced. When the diverting valve 295 is positioned at position A, and connects the insufflation/sensing conduit 118 to the conduits 251, 253 of the tube set 250 and thus to the surgical devices 231, 233, the insufflator subunit 121 is permitted to sense the abdominal pressure. In position A, output from the pump 111 enters the diverting valve 295 and is returned to the pump 111 immediately by mutually connecting the supply conduit 114 and return conduit 112 under such circumstances. This configuration allows the pump 111 to continue running during sensing and thus avoids any power spikes which might occur if stopping and restarting of the pump 111.

If the system 200 is set to a suitable mode (such as combined smoke evacuation and insufflation), when the insufflator subunit 121 is finished sensing the abdominal pressure, the diverting valve 295 is switched from position A, to Position B, in order to connect the supply conduit 114 to a corresponding conduit 251 of the tube set 250, and the return conduit 112 to a corresponding conduit 253 of the tube set 250. In position B, the insufflator conduit 118 is connected to the return conduit 112, permitting addition of insufflation gas into the system 200 through the return conduit 112. Concurrently, the insufflator subunit 121 can be set to insufflating mode only, therefore only adding gas to the system 200 and not sensing pressures.

While in position B, the diverting valve 295 permits delivery and return of fluid therethrough, between the pump 111 and filters 216, and the surgical devices 231, 233, and therefore gas exchange with, and filtration of, insufflation gasses from the surgical cavity 190.

If the pump and tube volume are considered as one controlled volume in conjunction with the volume of the surgical cavity 190, the function of the insufflator subunit 121 alone-switching from sensing to supplying carbon dioxide-is performed as in conventional surgical insufflators, in accordance with a preferred aspect.

Accordingly, as described above, in the system 200 of Figure 2, smoke evacuation and filtration is only performed when the diverting valve 295 permits the insufflator control 121 to provide gas to the surgical cavity 190. In such an arrangement, toggling to and from smoke evacuation/filtration and pressure sensing can be configured as either a normally sensing mode, or as a normally filtering mode, as desired or required. A normally sensing mode is likely to be preferred over a normally filtering mode, as monitoring of abdominal pressures is typically a priority.

As illustrated, position C of the diverting valve 295 permits the system 200 to be operated in a recirculation mode, the recirculation mode being suitable for providing sufficient pressures and flow rates to drive surgical access devices such as those described in U.S. Patent Publication Number 2007/0088275, as well as in U.S. Patent Application Serial Number 61/104,448, filed October 10, 2008, and/or those described in U.S. Patent Numbers 7,182,752, 7,285,112, 7,413,559 or 7,338,473, for example. In such a mode, a single tube of three lumens is typically provided, one lumen being in fluid communication with each of the supply conduit 112, return conduit 114 and the insufflation conduit 118.

For the purposes of explanation and illustration, and not limitation, a schematic illustration of an exemplary embodiment of a surgical gas delivery system in accordance with still another aspect of the invention is shown in Fig. 3 and is designated generally by reference character 300.

The functionality of components described above in connection with the systems 100 and 200 of Figures 1 and 2, respectively are the same as the corresponding components of the system 300 of Figure 3, unless otherwise specified.

As with the system 200 of Figure 2, the system 300 of Figure 3 utilizes two separate fluid conduits 251, 253 to surgical devices 231, 233, which are in fluid communication with the surgical cavity 190. Instead of a single insufflation/sense conduit 118, in the system 200 of Figure 2, a separate pressure sense conduit 318a and an insufflation fluid conduit 318b are provided. The pressure sense and insufflation fluid conduits 318a, 318b are provided in connection with the insufflation subunit 121, through one or more filters 216, and into a diverting valve 395. A supply conduit 114 and return conduit 112 provided in connection with the pump 111 are also fed into the diverting valve 395. This arrangement permits continuous addition of insufflation gas from the supply 140, if necessary.

As illustrated, the diverting valve 395 is provided with three positions, A, B and C. As illustrated in Figure 3, in valve 395 position C, the pressure sense conduit 318a and insufflation fluid conduit 318b are connected respectively to the two external fluid conduits 253, 251, to respective surgical devices (e.g. 233, 231), pressure sensing being accomplished through one surgical device (e.g. 233), while flow of insufflation gas is carried through the other conduit (e.g. 251) to the other surgical device (e.g., 231). Also in position C, the supply 114 and return 112 conduits are placed in fluid communication, bypassing the surgical devices 231, 233. Thus, the pump 111 may continue running and be re-activated in the system 300 by change in position of the valve 395. In this manner, power spikes are avoided with repeated starting and stopping of the pump 111.

As illustrated, the diverting valve 395 is provided integrally, within the control unit 310, behind filters 216, as schematically illustrated by placement of the broken line, referenced by element number 310.

When the insufflator control 121 is not sensing, the diverting valve 395 is positioned in position B, in which the conduits 251, 253 and surgical devices 231, 233 are placed in fluid connection, through the diverting valve 395, with the supply conduit 114 and return conduit 112, and thus with the pump 111, in order to provide smoke removal function, for example. With valve 395 in position B, the pressure sense conduit 318a terminates at the valve 395. In position B, supply of insufflation fluid can be provided by the insufflation subunit 121, through the insufflation conduit 318b, to the return line 112 to the pump 111, by way of the diverting valve 395, permitting continuous addition of insufflation gas to the system 300, if necessary. By providing insufflation gas to the return line 112 to the pump 111, insufflation gas is injected into the controlled volume of the abdomen through the return line 112, to the pump 111. Alternatively, if so-desired, addition of insufflation gas can be provided on the supply conduit 114 side of the pump 111.

In accordance with the invention, it is conceived that the rate of flow, which can be controlled by a user, is achieved, in one aspect, by the back-pressure control valve 113, which can be embodied as an electro-mechanical valve to enable interface with an electronic control system, for example.

In general, flow to and from the surgical devices 231, 235 for smoke evacuation/filtration functions will not solely affect pressure in the surgical cavity 190, because absent addition of gas by the insufflation subunit 121, only insufflation gas removed from the abdomen will be returned to the abdomen, and at the same flow rate. Otherwise, the pump 111 would become "starved."

As illustrated, position A of the diverting valve 395 permits the system 300 to be operated in a recirculation mode, the recirculation mode being suitable for providing sufficient pressures and flow rates to drive surgical access devices such as those described in U.S. Patent Publication Number 2007/0088275, as well as in U.S. Patent Application Serial Number 61/104,448, filed October 10, 2008, and/or those described in U.S. Patent Numbers 7,182,752, 7,285,112, 7,413,559 or 7,338,473, for example. In such a mode, a single tube of three lumens is typically provided, one lumen being in fluid communication with each of the supply conduit 112, return conduit 114 and the insufflation conduit 118.

In accordance with the invention, it is conceived that the rate of flow for smoke evacuation may be considerably less than the amount of flow used to power surgical access devices, such as those described in U.S. Pub. No. 2007/0088275 and US 61/104,448, filed October 10, 2008, for example. For example, if flow rates are excessively high, gas turbulence in the surgical cavity 190 may occur. Accordingly, integral flow restriction elements provided in connection with the tube sets 150, 250 or filter(s) 16 may be necessary to help reduce filtered gas flow.

## Claims

1. A multimodal surgical gas delivery system comprising:
- a fluid pump (111) adapted and configured to circulate insufflation fluid through the system;
- a supply conduit (114) in fluid communication with an output of the fluid pump (111) and configured and adapted for delivering pressurized insufflation fluid to an output port (183) of a control unit (210; 310);
- a return conduit (112) in fluid communication with an input of the fluid pump (111) for delivering insufflation fluid to the fluid pump (111) and configured and adapted for returning insufflation fluid to an input port (181) of the control unit (110);
- an adjustable back-pressure control valve (113) in fluid communication with the supply conduit (114) and the return conduit (112), the back-pressure control valve (113) being adapted and configured to respond to a supply conduit pressure exceeding a set pressure by opening and directing fluid from the supply conduit (114) to the return conduit (112);
- an insufflation control (121) for controlling addition of insufflation fluid into the system, from an insufflation gas source (140);
- an insufflation conduit (118) for delivering insufflation gas to the system from the insufflation control (121);
- a control panel configured and adapted to permit a user to select a mode of the multimodal surgical gas delivery system;
- a conduit set (250), adapted and configured to connect to the supply, return and insufflation conduits (114, 112, 118), and to a plurality of surgical devices (231, 233) in fluid communication with the surgical cavity,
- a pressure sensor (117), adapted and configured to sense pressure of a surgical cavity (190) through the insufflation conduit (118); and
- a dump valve (115) in fluid communication with the supply conduit (114) adapted and configured to release fluid from the system when an over-pressure condition is sensed,
**characterized by**
a switching valve (295; 395) in connection with the supply, return and insufflation conduits (114, 112, 118), the switching valve (295; 395) being configured and adapted to divert the insufflation conduit (118) between fluid connection with one or more of the surgical devices (231, 233), and the return conduit (112) to the fluid pump (111).

2. The system of claim 1, wherein the insufflation conduit (118) serves as a conduit for detecting abdominal pressure.

3. The system of claim 1, further comprising a conduit (318a) for detecting abdominal pressure, separate from the insufflation conduit (118).

## Patentansprüche

1. Multimodales chirurgisches Gaszufuhrsystem, umfassend:
- eine Fluidpumpe (111), die angepasst und eingerichtet ist, um ein Insufflationsfluid durch das System zu zirkulieren;
- eine Zufuhrleitung (114) in Fluidverbindung mit einem Ausgang der Fluidpumpe (111) und die zum Zuführen eines unter Druck stehenden Insufflationsfluids zu einem Ausgangsanschluss (183) einer Steuereinheit (210; 310) eingerichtet und angepasst ist;
- eine Rückführleitung (112) in Fluidverbindung mit einem Eingang der Fluidpumpe (111) zum Zuführen eines Insufflationsfluids zur Fluidpumpe (111) und die zum Zurückführen eines Insufflationsfluids zu einem Eingangsanschluss (181) der Steuereinheit (110) eingerichtet und angepasst ist;
- ein einstellbares Gegendruckregelventil (113) in Fluidverbindung mit der Zufuhrleitung (114) und der Rückführleitung (112), wobei das Gegendruckregelventil (113) angepasst und eingerichtet ist, um auf einen Zufuhrleitungsdruck, der einen eingestellten Druck überschreitet, durch Öffnen und Leiten eines Fluids von der Zufuhrleitung (114) zur Rückführleitung (112) zu reagieren;
- eine Insufflationssteuerung (121) zum Steuern einer Zugabe eines Insufflationsfluids von einer Insufflationsgasquelle (140) in das System;
- eine Insufflationsleitung (118) zum Zuführen eines Insufflationsgas von der Insufflationssteuerung (121) zum System;
- ein Bedienfeld, das eingerichtet und angepasst ist, um einem Benutzer zu ermöglichen, einen Modus des multimodalen chirurgischen Gaszufuhrsystems auszuwählen;
- einen Leitungssatz (250), der zum Verbinden mit den Zufuhr-, Rückführ- und Insufflationsleitungen (114, 112, 118) angepasst und eingerichtet ist, und um mit einer Mehrzahl von chirurgischen Vorrichtungen (231, 233) in Fluidverbindung mit dem chirurgischen Hohlraum zu stehen,
- einen Drucksensor (117), der angepasst und eingerichtet ist, um einen Druck eines chirurgischen Hohlraums (190) durch die Insufflationsleitung (118) zu erfassen; und
- ein Ablassventil (115) in Fluidverbindung mit der Zufuhrleitung (114), das angepasst und eingerichtet ist, um ein Fluid aus dem System abzulassen, wenn ein Überdruckzustand erfasst wird,
- **gekennzeichnet durch**
- ein Umschaltventil (295; 395) in Verbindung mit den Zufuhr-, Rückführ- und Insufflationsleitungen (114, 112, 118), wobei das Umschaltventil (295; 395) eingerichtet und angepasst ist, um die Insufflationsleitung (118) zwischen einer Fluidverbindung mit einer oder mehreren der chirurgischen Vorrichtungen (231, 233) und die Rückführleitung (112) zur Fluidpumpe (111) umzuschalten.

2. System nach Anspruch 1, wobei die Insufflationsleitung (118) als Leitung zum Erfassen eines Abdominaldrucks dient.

3. System nach Anspruch 1, das ferner eine Leitung (318a) zum Erfassen des Abdominaldrucks, getrennt von der Insufflationsleitung (118), aufweist.

## Revendications

1. Système multimodal de distribution de gaz chirurgical comprenant :
- une pompe à fluide (111) adaptée et configurée pour faire circuler un fluide d'insufflation à travers le système ;
- un conduit d'alimentation (114) en communication fluidique avec une sortie de la pompe à fluide (111) et configuré et adapté pour distribuer un fluide d'insufflation sous pression à un orifice de sortie (183) d'une unité de commande (210 ; 310) ;
- un conduit de retour (112) en communication fluidique avec une entrée de la pompe à fluide (111) pour distribuer un fluide d'insufflation à la pompe à fluide (111) et configuré et adapté pour renvoyer un fluide d'insufflation à un orifice d'entrée (181) de l'unité de commande (110) ;
- une soupape de commande de contre-pression réglable (113) en communication fluidique avec le conduit d'alimentation (114) et le conduit de retour (112), la soupape de commande de contre-pression (113) étant adaptée et configurée pour répondre à une pression de conduit d'alimentation supérieure à une pression de consigne par l'ouverture et l'orientation d'un fluide du conduit d'alimentation (114) au conduit de retour (112) ;
- une commande d'insufflation (121) pour commander un ajout de fluide d'insufflation dans le système, à partir d'une source de gaz d'insufflation (140) ;
- un conduit d'insufflation (118) pour distribuer un gaz d'insufflation au système à partir de la commande d'insufflation (121) ;
- un panneau de commande configuré et adapté pour permettre à un utilisateur de sélectionner un mode du système multimodal de distribution de gaz chirurgical ;
- un ensemble de conduits (250), adapté et configuré pour être relié aux conduits d'alimentation, de retour et d'insufflation (114, 112, 118), et à une pluralité de dispositifs chirurgicaux (231, 233) en communication fluidique avec la cavité chirurgicale,
- un capteur de pression (117), adapté et configuré pour détecter une pression d'une cavité chirurgicale (190) à travers le conduit d'insufflation (118) ; et
- une soupape de décharge (115) en communication fluidique avec le conduit d'alimentation (114) adaptée et configurée pour libérer un fluide du système lorsqu'une surpression est détectée,
**caractérisé par**
une soupape de commutation (295 ; 395) en liaison avec les conduits d'alimentation, de retour et d'insufflation (114, 112, 118), la soupape de commutation (295 ; 395) étant configurée et adaptée pour faire dévier le conduit d'insufflation (118) entre une liaison fluidique avec un ou plusieurs des dispositifs chirurgicaux (231, 233), et le conduit de retour (112) à la pompe à fluide (111).

2. Système de la revendication 1, dans lequel le conduit d'insufflation (118) sert de conduit pour détecter une pression abdominale.

3. Système de la revendication 1, comprenant en outre un conduit (318a) pour détecter une pression abdominale, séparé du conduit d'insufflation (118).
